# EUROPEAN PATENT APPLICATION

(11) **EP 4 483 869 A1**
(43) Date of publication of application: **01.01.2025**
(21) Application number: 24185202.9
(22) Date of filing: 28.06.2024
(51) Int. Cl.: A61K 31/215, A61K 31/225, A61K 31/23, A23K 10/00, A23K 20/105, A61P 3/00, A61P 5/00, A61P 9/00, A61P 25/00, A61P 29/00

(54) **KETONE ESTERS FOR USE IN INDUCING KETOSIS IN NON-HUMAN MAMMALS**

(30) Priority: 29.06.2023 US 202363510982 P
(71) Applicant: BOPZ nutrition Limited, IM8 1GB Ramsey (IM); Buck Institute for Research on Aging, Novato, CA 94945 (US)
(72) Inventor: NEVIES, Yehudah, London, NW11 6NG (GB); STUBBS, Brianna Jane, Richmond, 94801 (US); WEATHERLEY, Andrew, deceased (GB)
(74) Representative: Finnegan Europe LLP

(57) **Abstract**

The disclosure provides methods of inducing ketosis in a non-human mammal in need thereof, comprising administering to the non-human mammal an effective amount of a compound of Formula I, wherein the amount is from 125 mg/kg to 500 mg/kg.

## Description

The disclosure relates to methods for inducing ketosis in non-human mammals.

Ketosis is a metabolic state in which the levels of ketone bodies in an animal increase. In some embodiments, said increase occurs in the brain of the animal.

Ketosis may be characterized by increased levels of ketone bodies compared to a resting metabolic state.

Studies in humans indicated that ketone concentration correlates with efficacy for cognitive improvements (Fortier et al. (2021) A ketogenic drink improves cognition in mild cognitive impairment: Results of a 6-month RCT, Alzheimer's & dementia: the journal of the Alzheimer's Association, 17(3), pp. 543-52).

Ketosis may be assessed quantitatively by measuring plasma beta-hydroxybutyrate (BHB) concentrations. Methods of measuring plasma BHB concentrations are known in the art. Non-limiting exemplary methods for measuring plasma BHB concentrations are provided in US 10,562,839, which is hereby incorporated by reference in its entirety. Plasma BHB levels may be determined by a colorimetric enzymatic assay (Stanbio Laboratory 2440-058).

Ketosis may be induced by a ketogenic diet. A ketogenic diet may comprise high amounts of fat, moderate amounts of protein, and very low amounts of carbohydrate. For example, a ketogenic diet may comprise 80% high fat food, 15% leafy vegetable matter, and up to 5% complex carbohydrate.

Compositions comprising medium-chain triglycerides (MCTs) are known in the prior art to induce ketosis in canines. One prior study demonstrated MCTs induce ketosis in canines with a Cₘₐₓ for BHB of 0.06 mmol (Pan et al. (2010) Dietary supplementation with medium-chain TAG has long-lasting cognition-enhancing effects in aged dogs, British Journal of Nutrition, 103(12), pp. 1746-1754). Another prior study, looking at long-term administration of MCTs, demonstrated that even after continued administration of MCTs for two months, BHB concentrations only reached an average of about 0.25 mmol (Studzinski et al. (2008) Induction of ketosis may improve mitochondrial function and decrease steady-state amyloid-β precursor protein (APP) levels in the aged dog, Brain Research, 1226, pp. 209-217).

A need exists for methods of inducing ketosis in non-human mammals to higher levels than those achieved with MCTs.

The disclosure is directed to improved methods of inducing ketosis in non-human mammals in need thereof.

### SUMMARY

The disclosure is directed to methods of inducing ketosis in non-human mammals.

In some embodiments, the disclosure is directed to a method of inducing ketosis in a non-human mammal in need thereof, comprising administering to the non-human mammal an effective amount of a compound of Formula I: 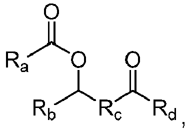 wherein: Rₐ is unsubstituted or substituted C₄₋₃₀ alkyl; R_{b} is H, C₁₋₆ alkyl, or substituted alkyl; R_{c} is CHzHzO or CH₂; and R_{d} is unsubstituted or substituted C₄₋₃₀ alkyl, or unsubstituted or substituted O-C₄₋₃₀ alkyl. In some embodiments, the amount of the compound is from 125 mg/kg to 500 mg/kg.

In some embodiments, the compound is a compound of Formula II: 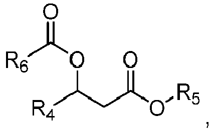 wherein: R₄ is H, C₁₋₆ alkyl, or substituted alkyl; and R₅ and R₆ are independently unsubstituted or substituted C₄₋₃₀ alkyl.

In some embodiments, the compound is a compound of Formula III: 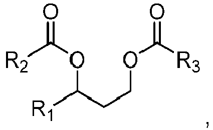 wherein: R₁ is H, C₁₋₆ alkyl, or substituted alkyl; and R₂ and R₃ are independently unsubstituted or substituted C₄₋₃₀ alkyl.

In some embodiments, the compound is selected from the group consisting of hexanoyl-ethyl-3-hydroxybutyrate, octanoyl-ethyl-3-hydroxybutyrate, hexanoyl-hexyl-3-hydroxybutyrate, bis-hexanoyl-1,3-butanediol, and bis-octanoyl-1,3-butanediol.

In some embodiments, the amount of the compound is administered as a food supplement.

In some embodiments, the amount is 125 mg/kg.

In some embodiments, the amount is 250 mg/kg.

In some embodiments, the amount is 500 mg/kg.

In some embodiments, the amount of the compound is administered at least once per day.

In some embodiments, the non-human mammal is administered a composition comprising the compound.

In some embodiments, wherein the non-human mammal is administered a composition comprising the compound, the composition further comprises one or more compounds or components of a ketogenic diet.

In some embodiments, the non-human mammal is a domestic animal.

In some embodiments, the domestic animal is a companion animal.

In some embodiments, the companion animal is a canine or feline.

In some embodiments, the domestic animal is an equine.

The disclosure is further described in the following enumerated embodiments.

### Enumerated embodiments

1. A method of inducing ketosis in a non-human mammal in need thereof, comprising administering to the non-human mammal an effective amount of a compound of Formula I: 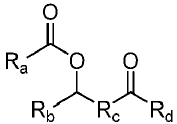 wherein:
   Rₐ is unsubstituted or substituted C₄₋₃₀ alkyl;
   R_{b} is H, C₁₋₆ alkyl, or substituted alkyl;
   R_{c} is CHzHzO or CH₂; and
   R_{d} is unsubstituted or substituted C₄₋₃₀ alkyl, or unsubstituted or substituted O-C₄₋₃₀ alkyl;
   wherein the amount is from 125 mg/kg to 500 mg/kg.
2a. A method of embodiment 1, wherein Rₐ is unsubstituted C₄₋₃₀ alkyl.
2b. A method of embodiment 1, wherein Rₐ is substituted C₄₋₃₀ alkyl.
3a. A method of embodiment 1 or embodiment 2, wherein R_{b} is H.
3b. A method of embodiment 1 or embodiment 2, wherein R_{b} is C₁₋₆ alkyl.
3c. A method of embodiment 1 or embodiment 2, wherein R_{b} is substituted alkyl.
4a. A method of any of embodiments 1-3, wherein R_{c} is CHzHzO.
4b. A method of any of embodiments 1-3, wherein R_{c} is CH₂.
5a. A method of any of embodiments 1-4, wherein R_{d} is substituted C₄₋₃₀ alkyl.
5b. A method of any of embodiments 1-4, wherein R_{d} is unsubstituted C₄₋₃₀ alkyl.
5c. A method of any of embodiments 1-4, wherein R_{d} is substituted O-C₄₋₃₀ alkyl.
5d. A method of any of embodiments 1-4, wherein R_{d} is unsubstituted O-C₄₋₃₀ alkyl.
6. A method of any of embodiments 1-5, wherein the compound is a compound of Formula II: 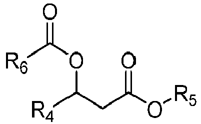 wherein:
   R₄ is H, C₁₋₆ alkyl, or substituted alkyl; and
   R₅ and R₆ are independently unsubstituted or substituted C₄₋₃₀ alkyl.
7a. The method of embodiment 6, wherein R₄ is H.
7b. The method of embodiment 6, wherein R₄ is C₁₋₆ alkyl.
7c. The method of embodiment 6, wherein R₄ is substituted alkyl.
8a. The method of embodiment 6 or embodiment 7, wherein R₅ is unsubstituted C₄₋₃₀ alkyl.
8b. The method of embodiment 6 or embodiment 7, wherein R₅ is substituted C₄₋₃₀ alkyl.
8c. The method of embodiment 6 or embodiment 7, wherein R₅ is unsubstituted C₂₋₈ alkyl.
8d. The method of embodiment 6 or embodiment 7, wherein R₅ is substituted C₂₋₈ alkyl.
9a. The method of any of embodiments 6-8, wherein R₆ is unsubstituted C₄₋₃₀ alkyl.
9b. The method of any of embodiments 6-8, wherein R₆ is substituted C₄₋₃₀ alkyl.
9c. The method of any of embodiments 6-8, wherein R₆ is unsubstituted C₆₋₈ alkyl.
9d. The method of any of embodiments 6-8, wherein R₆ is substituted C₆₋₈ alkyl.
10. The method of embodiments 1-5, wherein the compound is a compound of Formula III: 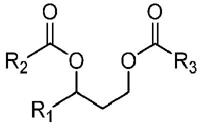 wherein:
   R₁ is H, C₁₋₆ alkyl, or substituted alkyl; and
   R₂ and R₃ are independently unsubstituted or substituted C₄₋₃₀ alkyl.
11a. The method of embodiment 10, wherein R₁ is H.
11b. The method of embodiment 10, wherein R₁ is C₁₋₆ alkyl.
11c. The method of embodiment 10, wherein R₁ is substituted alkyl.
12a. The method of embodiment 10 or embodiment 11, wherein R₂ is unsubstituted C₄₋₃₀ alkyl.
12b. The method of embodiment 10 or embodiment 11, wherein R₂ is substituted C₄₋₃₀ alkyl.
12c. The method of embodiment 10 or embodiment 11, wherein R₂ is unsubstituted C₆₋₈ alkyl.
12d. The method of embodiment 10 or embodiment 11, wherein R₂ is substituted C₆₋₈ alkyl.
13a. The method of any of embodiments 10-12, wherein R₃ is unsubstituted C₄₋₃₀ alkyl.
13b. The method of any of embodiments 10-12, wherein R₃ is substituted C₄₋₃₀ alkyl.
13c. The method of any of embodiments 10-12, wherein R₃ is unsubstituted C₂₋₈ alkyl.
13d. The method of any of embodiments 10-12, wherein R₃ is substituted C₂₋₈ alkyl.
14a. The method of any of embodiments 1-5, wherein the compound is hexanoyl-ethyl-3-hydroxybutyrate.
14b. The method of any of embodiments 1-5, wherein the compound is octanoyl-ethyl-3-hydroxybutyrate.
14c. The method of any of embodiments 1-5, wherein the compound is hexanoyl-hexyl-3-hydroxybutyrate.
14d. The method of any of embodiments 1-5, wherein the compound is bis-hexanoyl-1,3-butanediol.
14e. The method of any of embodiments 1-5, wherein the compound is bis-octanoyl-1,3-butanediol.
15. The method of any preceding embodiment, wherein the amount of the compound is administered as a food supplement.
16. The method of any preceding embodiment, wherein the amount is 125 mg/kg.
17. The method of any of embodiments 1-15, wherein the amount is 250 mg/kg.
18. The method of any of embodiments 1-15, wherein the amount is 500 mg/kg.
19. The method of any preceding embodiment, wherein the amount of the compound is administered at least once per day.
20. The method of any preceding embodiment, wherein the non-human mammal is administered a composition comprising the compound.
21. The method of embodiment 20, wherein the composition further comprises one or more compounds or components of a ketogenic diet.
22. The method of any preceding embodiment, wherein the non-human mammal is a domestic animal.
23. The method of embodiment 22, wherein the domestic animal is a canine, a feline, or an equine.
24. The method of embodiment 22, wherein the domestic animal is a companion animal.
25. The method of embodiment 24, wherein the companion animal is a canine or a feline.
26a. The method of embodiment 23 or embodiment 25, wherein the animal is a canine.
26b. The method of embodiment 23 or embodiment 25, wherein the animal is a feline.
27. The method of embodiment 23, wherein the domestic animal is an equine.
28. A non-human mammal food supplement, food, or food additive comprising a compound of Formula I: 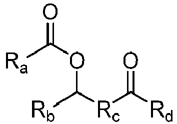 wherein:
   Rₐ is unsubstituted or substituted C₄₋₃₀ alkyl;
   R_{b} is H, C₁₋₆ alkyl, or substituted alkyl;
   R_{c} is CHzHzO or CH₂; and
   R_{d} is unsubstituted or substituted C₄₋₃₀ alkyl, or unsubstituted or substituted O-C₄₋₃₀ alkyl;
   wherein the amount is from 125 mg/kg to 500 mg/kg.
29a. The food supplement, food, or food additive of embodiment 28, wherein Rₐ is unsubstituted C₄₋₃₀ alkyl.
29b. The food supplement, food, or food additive of embodiment 28, wherein Rₐ is substituted C₄₋₃₀ alkyl.
30a. The food supplement, food, or food additive of embodiment 28 or embodiment 29, wherein R_{b} is H.
30b. The food supplement, food, or food additive of embodiment 28 or embodiment 29, wherein R_{b} is C₁₋₆ alkyl.
30c. The food supplement, food, or food additive of embodiment 28 or embodiment 29, wherein R_{b} is substituted alkyl.
31a. The food supplement, food, or food additive embodiments 28-30, wherein R_{c} is CHzHzO.
31b. The food supplement, food, or food additive embodiments 28-30, wherein R_{c} is CH₂.
32a. The food supplement, food, or food additive embodiments 28-31, wherein R_{d} is substituted C₄₋₃₀ alkyl.
32b. The food supplement, food, or food additive embodiments 28-31, wherein R_{d} is unsubstituted C₄₋₃₀ alkyl.
32c. The food supplement, food, or food additive embodiments 28-31, wherein R_{d} is substituted O-C₄₋₃₀ alkyl.
32d. The food supplement, food, or food additive embodiments 28-31, wherein R_{d} is unsubstituted O-C₄₋₃₀ alkyl.
33. The food supplement, food, or food additive embodiments 28-32, wherein the compound is a compound of Formula II: 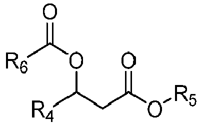 wherein:
   R₄ is H, C₁₋₆ alkyl, or substituted alkyl; and
   R₅ and R₆ are independently unsubstituted or substituted C₄₋₃₀ alkyl.
34a. The food supplement, food, or food additive of embodiment 33, wherein R₄ is H.
34b. The food supplement, food, or food additive of embodiment 33, wherein R₄ is C₁₋₆ alkyl.
34c. The food supplement, food, or food additive of embodiment 33, wherein R₄ is substituted alkyl.
35a. The food supplement, food, or food additive of embodiment 33 or embodiment 34, wherein R₅ is unsubstituted C₄₋₃₀ alkyl.
35b. The food supplement, food, or food additive of embodiment 33 or embodiment 34, wherein R₅ is substituted C₄₋₃₀ alkyl.
35c. The food supplement, food, or food additive of embodiment 33 or embodiment 34, wherein R₅ is unsubstituted C₂₋₈ alkyl.
35d. The food supplement, food, or food additive of embodiment 33 or embodiment 34, wherein R₅ is substituted C₂₋₈ alkyl.
36a. The food supplement, food, or food additive of embodiments 33-35, wherein R₆ is unsubstituted C₄₋₃₀ alkyl.
36b. The food supplement, food, or food additive of embodiments 33-35, wherein R₆ is substituted C₄₋₃₀ alkyl.
36c. The food supplement, food, or food additive of embodiments 33-35, wherein R₆ is unsubstituted C₆₋₈ alkyl.
36d. The food supplement, food, or food additive of embodiments 33-35, wherein R₆ is substituted C₆₋₈ alkyl.
37. The food supplement, food, or food additive of embodiments 28-32, wherein the compound is a compound of Formula III: 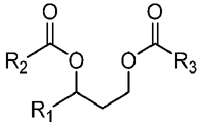 wherein:
   R₁ is H, C₁₋₆ alkyl, or substituted alkyl; and
   R₂ and R₃ are independently unsubstituted or substituted C₄₋₃₀ alkyl.
38a. The food supplement, food, or food additive of embodiment 37, wherein R₁ is H.
38b. The food supplement, food, or food additive of embodiment 37, wherein R₁ is C₁₋₆ alkyl.
38c. The food supplement, food, or food additive of embodiment 37, wherein R₁ is substituted alkyl.
39a. The food supplement, food, or food additive of embodiment 37 or embodiment 38, wherein R₂ is unsubstituted C₄₋₃₀ alkyl.
39b. The food supplement, food, or food additive of embodiment 37 or embodiment 38, wherein R₂ is substituted C₄₋₃₀ alkyl.
39c. The food supplement, food, or food additive of embodiment 37 or embodiment 38, wherein R₂ is unsubstituted C₆₋₈ alkyl.
39d. The food supplement, food, or food additive of embodiment 37 or embodiment 38, wherein R₂ is substituted C₆₋₈ alkyl.
40a. The food supplement, food, or food additive of embodiments 37-39, wherein R₃ is unsubstituted C₄₋₃₀ alkyl.
40b. The food supplement, food, or food additive of embodiments 37-39, wherein R₃ is substituted C₄₋₃₀ alkyl.
40c. The food supplement, food, or food additive of embodiments 37-39, wherein R₃ is unsubstituted C₂₋₈ alkyl.
40d. The food supplement, food, or food additive of embodiments 37-39, wherein R₃ is substituted C₂₋₈ alkyl.
41a. The food supplement, food, or food additive of embodiments 28-32, wherein the compound is hexanoyl-ethyl-3-hydroxybutyrate.
41b. The food supplement, food, or food additive of embodiments 28-32, wherein the compound is octanoyl-ethyl-3-hydroxybutyrate.
41c. The food supplement, food, or food additive of embodiments 28-32, wherein the compound is hexanoyl-hexyl-3-hydroxybutyrate.
41d. The food supplement, food, or food additive of embodiments 28-32, wherein the compound is bis-hexanoyl-1,3-butanediol.
41e. The food supplement, food, or food additive of embodiments 28-32, wherein the compound is bis-octanoyl-1,3-butanediol.
42. The food supplement, food, or food additive of embodiments 28-41, wherein the amount is 125 mg/kg.
43. The food supplement, food, or food additive of embodiments 28-41, wherein the amount is 250 mg/kg.
44. The food supplement, food, or food additive of embodiments 28-41, wherein the amount is 500 mg/kg.
45. The food supplement, food or food additive of embodiments 28-44, wherein the food supplement, food or food additive is for administration at least once per day.
46. The food supplement, food, or food additive of embodiments 28-45, further comprising one or more compounds or components of a ketogenic diet.
47. The food supplement, food, or food additive of embodiments 28-46, wherein the non-human mammal is a domestic animal.
48. The food supplement, food, or food additive of embodiment 47, wherein the domestic animal is a canine, a feline, or an equine.
49. The food supplement, food, or food additive of embodiment 47, wherein the domestic animal is a companion animal.
50. The food supplement, food, or food additive of embodiment 49, wherein the companion animal is a canine or a feline.
51a. The food supplement, food, or food additive of embodiment 48 or embodiment 50, wherein the animal is a canine.
51b. The food supplement, food, or food additive of embodiment 48 or embodiment 50, wherein the animal is a feline.
52. The food supplement, food, or food additive of embodiment 48, wherein the domestic animal is an equine.

### BRIEF DESCRIPTION OF DRAWING(S)

The drawings are for illustration purposes only and do not provide any limitation on the disclosure.

Figures 1A and 1B illustrate the sampling periods in fasted canines following administration of compounds for inducing ketosis. Figure 1A illustrates sampling periods following administration of MCTs. Figure 1B illustrates sampling periods following administration of 250 mg/kg of a compound of the disclosure.

Figures 2A-2D illustrate the sampling periods in fed canines following administration of compounds for inducing ketosis. Figure 2A illustrates sampling periods following administration of 250 mg/kg of a compound of the disclosure. Figure 2B illustrates sampling periods following administration of 500 mg/kg of a compound of the disclosure. Figure 2C illustrates sampling periods following administration of MCTs. Figure 2D illustrates sampling periods following administration of 125 mg/kg of a compound of the disclosure.

Figures 3A, 3B, 3C and 3D illustrate plasma BHB concentrations in fed canines following administration of compounds of the disclosure. Figure 3A illustrates BHB concentrations following administration of C8-BHB. Figure 3B illustrates BHB concentrations following administration of C8x2-BD. Figure 3C illustrates the raw data corresponding to Figure 3A. Figure 3D illustrates the raw data corresponding to Figure 3D.

Figure 4 illustrates plasma BHB concentrations in fed canines following administration of compounds for inducing ketosis.

Figures 5A and 5B illustrate statistical analysis for BHB concentrations following administration of compounds for inducing ketosis. Figure 5A illustrates Cₘₐₓ. Figure 5B illustrates AUC.

Figures 6A and 6B illustrate statistical analysis for individual canines following administration of compounds for inducing ketosis. Figure 6A illustrates raw data for individual canines. Figure 6B graphically depicts the data from Figure 6A.

Figure 7 illustrates statistical analysis for fasted individual canines following administration of compounds for inducing ketosis.

### DETAILED DESCRIPTION

The disclosure is directed to methods of inducing ketosis in non-human mammals.

As used herein, the terms "individual" and "subject" are used to refer to a non-human mammal. In some embodiments, the non-human mammal is a companion animal. Optionally, the non-human mammal is a canine or a feline. In some embodiments, the non-human mammal is an equine.

As used herein, an "effective amount" or "efficacious amount" refers to an amount of a compound that, when administered to a subject in need thereof, is sufficient to induce ketosis. In some embodiments, an "effective amount" or "efficacious amount" of a compound of the disclosure is 125 mg/kg to 500 mg/kg.

As used herein, the terms "treatment," "treating," and the like, refer to obtaining a desired pharmacologic and/or physiologic effect. The effect may be prophylactic. The effect may be the reduction of one or more symptoms.

As used herein, "inducing ketosis" refers to increasing the amount and/or concentration of one or more ketone bodies in the subject.

### Definition of Select Chemical Terminology

The nomenclature of certain compounds or substituents are used in their conventional sense, such as described in chemistry literature including but not limited to Loudon, Organic Chemistry, Fourth Edition, New York: Oxford University Press, 2002, pp. 360-361, 1084-1085; Smith and March, March's Advanced Organic Chemistry: Reactions, Mechanisms, and Structure, Fifth Edition, Wiley-Interscience, 2001.

Further definitions of select chemical terminology are provided in US 10,562,839, which is herein incorporated by reference in its entirety.

### Compounds

The methods of the disclosure comprise administering to a subject in need thereof an effective amount of a compound for inducing ketosis.

In some embodiments, the compound for inducing ketosis is a fatty acid beta-hydroxyester compound, a fatty acid ester of butanediol, and/or an acceptable salt thereof. In some embodiments, an effective salt of a compound means a salt that possesses the desired pharmacological activity of the parent compounds. In some embodiments of the disclosure, an acceptable salt of a compound induces ketosis following administration to a non-human mammal. In some embodiments, the acceptable salt is a pharmaceutically acceptable salt. Exemplary pharmaceutically acceptable salts are disclosed in US 10,562,839, which is hereby incorporated by reference in its entirety.

The compounds of the disclosure produce increased ketone body concentration in a subject. Increasing ketone bodies induces ketosis.

Without wishing to be bound by any particular theory, the compounds of the disclosure are thought to release, via ester hydrolysis, beta-hydroxybutyrate (BHB) or butanediol (BD). BHB and BD are thought to increase ketone body concentration in the subject, and provide a supplemental source of medium chain fatty acids that provide for sustained ketone body production by the subject. The compounds of the disclosure increase ketone body concentrations and induce ketosis.

In some embodiments, the compounds of the disclosure are fatty acid esters of BHB that hydrolyze, after administration to a non-human mammal, to BHB and medium chain fatty acids. The release of BHB provides for an increase in ketone body concentrations in the non-human mammal, and the hydrolyzed medium chain fatty acids provide a substrate for sustained physiological production of ketone bodies.

In some embodiments, the compounds of the disclosure are fatty acid esters of BD that hydrolyze, after administration to a non-human mammal, to BD and medium chain fatty acids. The release of BD provides for an increase in ketone body concentrations in the non-human mammal, and the hydrolyzed medium chain fatty acids provide a substrate for sustained physiological production of ketone bodies.

In some embodiments, the methods of the disclosure comprise administering to a subject in need thereof, an amount of a compound according to Formula I: 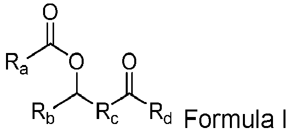 wherein:
Rₐ is unsubstituted or substituted C₄₋₃₀ alkyl;
R_{b} is H, C₁₋₆ alkyl, or substituted alkyl;
R_{c} is CHzHzO or CH₂; and
R_{d} is unsubstituted or substituted C₄₋₃₀ alkyl, or unsubstituted or substituted O-C₄₋₃₀ alkyl.

In some embodiments, the disclosure is directed to a compound according to Formula II: 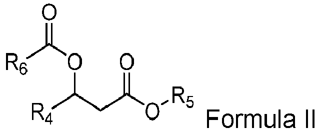 wherein:
R₄ is H or C₁₋₆ alkyl or substituted alkyl; and
R₅ and R₆ are independently unsubstituted or substituted C₄₋₃₀ alkyl.

In some embodiments, the disclosure is directed to a compound according to Formula III: 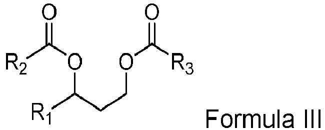 wherein:
R₁ is H or C₁₋₆ alkyl or substituted alkyl; and
R₂ and R₃ are independently unsubstituted or substituted C₄₋₃₀ alkyl.

In some embodiments, the disclosure is directed to a compound according to Formula II or Formula III, wherein R₂ or R₆ is unsubstituted or substituted C₆₋₈ alkyl.

In some embodiments, the disclosure is directed to a compound according to Formula II or Formula III, wherein R₃ or R₅ is unsubstituted or substituted C₂₋₈ alkyl.

In some embodiments, the compound of the disclosure according to Formula II is hexanoyl-ethyl-3-hydroxybutyrate (C6-BHB). In some embodiments, C6-BHB comprises the R enantiomer, the S enantiomer, or racemate. 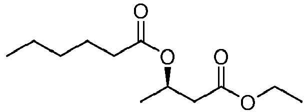 C6-BHB (R enantiomer)

In some embodiments, the compound of the disclosure according to Formula II is octanoyl-ethyl-3-hydroxybutyrate (C8-BHB). In some embodiments, C8-BHB comprises the R enantiomer, the S enantiomer, or racemate. 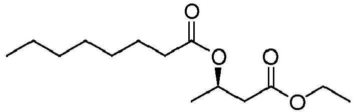 C8-BHB (R enantiomer)

In some embodiments, the compound of the disclosure according to Formula II is hexanoyl-hexyl-3-hydroxybutyrate (C6x2-BHB). In some embodiments, C6x2-BHB comprises the R enantiomer, the S enantiomer, or racemate. 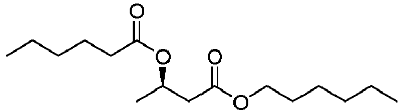 C6x2-BHB (R enantiomer)

In some embodiments, the compound of the disclosure according to Formula III is bis-hexanoyl-1,3-butanediol (C6x2-BD). In some embodiments, C6x2-BD comprises the R enantiomer, the S enantiomer, or racemate. 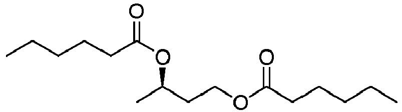 C6x2-BD (R enantiomer)

In some embodiments, the compound of the disclosure according to Formula III is bis-octanoyl-1,3-butanediol (C8x2-BD). In some embodiments, C6x2-BD comprises the R enantiomer, the S enantiomer, or racemate. 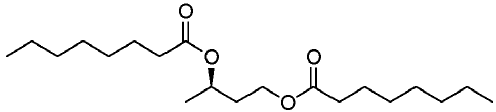 C8x2-BD (R enantiomer)

In an alternative embodiment, the compound of the disclosure is ethyl hexanoate (C6).

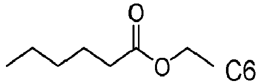

In a further alternative embodiment, the compound of the disclosure is beta hydroxy-butyrate (BHB). In some embodiments, BHB comprises the R enantiomer, the S enantiomer, or racemate. 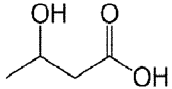 BHB

In a further alternative embodiment, the compound of the disclosure is hexyl hexanoate (C6x2). 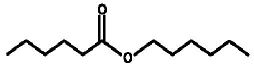 C6x2

In a further alternative embodiment, the compound of the disclosure is an S-ester. 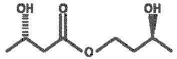 S-ester

### Inducing ketosis

In some embodiments of the disclosure, the induction of ketosis is identified by an increase in ketone bodies.

In some embodiments of the disclosure, the induction of ketosis is identified by an increase in one or more specific ketone bodies.

In some embodiments of the disclosure, the induction of ketosis is identified by an increase in BHB.

In some embodiments, the concentration of one or more ketone bodies is measured in plasma. Methods for obtaining plasma samples and measuring the concentrations of ketone bodies are known in the art.

In some embodiments, an "increase" is relative to baseline levels in a subject prior to administration of a compound to induce ketosis.

In some embodiments, an "increase" is relative to a resting metabolic state.

In some embodiments, an "increase" is relative to known, standard levels, in a subject. In some embodiments, the subject is a non-human mammal, optionally the subject is a companion animal such as a canine.

In some embodiments, wherein the subject is a canine, the baseline level of ketone in a fed subject is 0.018 to 0.024 mM.

In some embodiments, induction of ketosis is indicated by a Cₘₐₓ value of at least 0.05 mM following administration relative to baseline. Optionally, the Cₘₐₓ value is at least 0.1 mM, at least 0.5 mM, at least 0.6 mM, or at least 1.0 mM.

### Subject in need thereof

The methods of the disclosure are directed to inducing ketosis in a subject in need thereof, wherein the subject in need thereof is a non-human mammal.

In some embodiments, the non-human mammal is a domestic animal. In some embodiments, a domestic animal is any animal kept as a pet or to produce food. Exemplary domestic animals include canines (dogs), felines (cats), leporines (rabbits), assinines (donkeys), bovines (cows), ovines (sheep), porcines (pigs), equines (horses), and caprines (goats).

In some embodiments, the domestic mammal is an equine, a canine, or a feline.

In some embodiments, the non-human mammal is an equine.

In some embodiments, the non-human mammal is a companion animal. In some embodiments, a companion animal is a pet. Exemplary companion animals include canines and felines. Other companion animals are known in the art.

In some embodiments, the companion animal is a canine or feline.

In some embodiments, the companion animal is a canine.

In some embodiments, the companion animal is a feline.

In some embodiments, the subject in need thereof is an elderly subject. In some embodiments, the elderly subject in need thereof is in need of elderly support.

In some embodiments, the elderly subject is an elderly canine. In some embodiments, said elderly canine is at least 5 years old. In some embodiments, said canine is at least 6 years old, at least 7 years old, at least 8 years old, at least 9 years old, at least 10 years old, at least 11 years old, at least 12 years old, at least 13 years old, at least 14 years old, or at least 15 years old.

In some embodiments, the subject in need thereof is in need of metabolic benefits.

In some embodiments, metabolic benefits comprise reduced inflammation, improved fat burning, improved weight loss, blood sugar balancing, improved insulin sensitivity, suppression of ghrelin, reduced cravings, increased energy, longer-lasting energy, improved liver health, anti-ageing, improved cardiac output, improved cardiac function, and/or mitochondrial biogenesis.

In some embodiments, the subject in need thereof is in need of support for healthy ageing.

In some embodiments, healthy ageing comprises a reduction in conditions and/or behaviors associated with ageing. Conditions and/or behaviors associated with ageing in non-human mammals are known in the art. Exemplary conditions and/or behaviors associated with ageing in canines are provided in McKenzie et al. (2022) The phenotype of aging in the dog: how aging impacts the health and well-being of dogs and their caregivers, J. Am Vet. Med. Assoc., 260(9), pp. 963-970.

In some embodiments, inducing ketosis supports and/or enhances brain function in the subject.

In some embodiments, the subject in need thereof is in need of support and/or enhancement of brain function.

In some embodiments, enhanced brain function comprises replacing glucose with ketones as a substrate for brain energy production, reduced free radical production and/or increased antioxidant levels in the brain, decreased pro-inflammatory compounds in the brain, and/or the prevention of blood vessel wall thickening in the brain.

In some embodiments, inducing ketosis supports cognitive function. Optionally, inducing ketosis supports cognitive function in elderly canines.

In some embodiments, the subject in need thereof is a non-human mammal in need of support of cognitive function.

In some embodiments, cognitive function is the performance of mental processes, including perception, learning, memory, understanding, awareness, reasoning, judgement, and/or intuition. In some embodiments, a subject in need of support of cognitive function presents with reduced performance in one or more cognitive function. Tests for reduced cognitive function in are known in the art. Exemplary tests for reduced cognitive function in canines are disclosed in Chapagain et al. (2018) Cognitive Aging in Dogs, Gerontology, 64(2), pp. 165-171).

In some embodiments, inducing ketosis provides an alternative source of fuel for the brain.

In some embodiments, the subject in need thereof is a non-human mammal in need of an alternative source of fuel for the brain.

In some embodiments, an alternative source of fuel for the brain is a ketone and/or ketone body.

In some embodiments, inducing ketosis reduces seizure frequency. In some embodiments, inducing ketosis treats and/or prevents idiopathic epilepsy.

In some embodiments, the subject in need thereof is a non-human mammal with epilepsy. In some embodiments, the non-human mammal in need thereof has been diagnosed with epilepsy. In some embodiments, the epilepsy is idiopathic epilepsy.

In some embodiments, the subject in need thereof is a non-human mammal in need of cardiovascular support and/or treatment for one or more cardiovascular conditions.

In some embodiments, inducing ketosis treats a disease causing cognitive decline and/or dysfunction in a non-human mammal.

In some embodiments, the non-human mammal in need thereof is a mammal with a disease causing cognitive decline and/or dysfunction. In some embodiments, the non-human mammal in need thereof has been diagnosed with a disease causing cognitive decline and/or dysfunction.

In some embodiments, inducing ketosis treats canine cognitive dysfunction syndrome (CCDS).

In some embodiments, the canine in need thereof is a canine with CCDS. In some embodiments, the canine in need thereof has been diagnosed with CCDS.

### Dosage

The methods of the disclosure comprise administering to the subject in need thereof an amount of from 125 mg/kg to 500 mg/kg of a compound of the disclosure.

In some embodiments, mg/kg refers to milligrams (mg) of the compound, per kilogram (kg) of weight of the non-human mammal to which the compound is to be administered.

In some embodiments, the method of the disclosure comprises administering an amount of from 125 mg/kg to 250 mg/kg of a compound of the disclosure.

In some embodiments, the method of the disclosure comprises administering an amount of from 250 mg/kg to 500 mg/kg of a compound of the disclosure.

In some embodiments, the method comprises administering an amount of 125 mg/kg of a compound of the disclosure.

In some embodiments, the method comprises administering an amount of 250 mg/kg of a compound of the disclosure.

In some embodiments, the method comprises administering an amount of 500 mg/kg of a compound of the disclosure.

In some embodiments, the amount of the compound of the disclosure is administered at least once per day.

In some embodiments, the amount of the compound of the disclosure is administered at least twice per day, at least three times per day, or at least four times per day.

In some embodiments, the amount of the compound of the disclosure is divided among the two of more administrations per day.

In a non-limiting example, where the amount of the compound is 250 mg/kg and the compound is administered twice per day, the compound is administered in two amounts of 125 mg/kg.

In a further non-limiting example, where the amount of the compound is 500 mg/kg and the compound is administered four per day, the compound is administered in four amounts of 125 mg/kg.

### Synthesis

Compounds according to the disclosure can be made by methods known in the art.

Exemplary methods for synthesizing compounds according to the disclosure are provided in US10,562,839 and US10,647,658, which are hereby incorporated by reference in their entirety.

### Formulations and Administration

In some embodiments, the formulation comprising a compound of the disclosure comprises one or more carriers. Acceptable carriers for use with compounds of the disclosure comprise aqueous or non-aqueous solutions, suspensions, and emulsions. In some embodiments, the carrier is pharmaceutically acceptable. In some embodiments, the carrier is sterile. Pharmaceutically acceptable carriers are known in the art.

In some embodiments, the formulation comprising a compound of the disclosure comprises one or more excipients. Suitable excipients are known in the art. In some embodiments, the excipient is pharmaceutically acceptable. In some embodiments, the excipient is sterile.

In some embodiments, the compound of the disclosure is formulated for oral ingestion.

In some embodiments, the compound of the disclosure is formulated as an oral preparation. In some embodiments, the oral preparation is a tablet, powder, granule, or capsule.

Exemplary formulations are disclosed in US 10,562,839, which is hereby incorporated by reference in its entirety.

In some embodiments, the compound of the disclosure is formulated as an ingestible composition.

In some embodiments, the compound of the disclosure is formulated as a supplement.

In some embodiments, the disclosure is directed to a non-human mammal food supplement comprising a compound of the disclosure in an amount of from 125 mg/kg to 500 mg/kg.

In some embodiments, a supplement comprises one or more nutrients to increase the amount of said nutrient consumed by a subject. A supplement is provided in addition to food consumed by the subject. In some embodiments, a supplement is a pill, a capsule, a tablet, a powder, or a liquid. In some embodiments, the nutrient is a compound of the disclosure.

In some embodiments, the compound of the disclosure is formulated as a food.

In some embodiments, the disclosure is directed to a non-human mammal food comprising a compound of the disclosure in an amount of from 125 mg/kg to 500 mg/kg.

In some embodiments, the compound of the disclosure is formulated as a food additive.

In some embodiments, the disclosure is directed to a non-human mammal food additive comprising a compound of the disclosure in an amount of from 125 mg/kg to 500 mg/kg.

In some embodiments, a food additive is a composition added to a food source (e.g., pet food) to introduce specific nutrients and/or to increase the quantity of specific nutrients consumed by a subject. In some embodiments, the compound of the disclosure is the specific nutrient provided in the food additive.

In some embodiments, the compound of the disclosure is formulated as a food, a food additive, or a supplement that further comprises one or more compounds or components of a ketogenic diet.

In some embodiments, the food supplement, food, or food additive is a companion animal food supplement, food, or food additive.

In some embodiments, the food supplement, food, or food additive is a canine or feline food supplement, food, or food additive.

In some embodiments, the food supplement, food, or food additive is an equine food supplement, food, or food additive.

Compounds and components of a ketogenic diet are known in the art.

Exemplary ketogenic diets for canines and components thereof are described in PCT/US2001/018801, which is hereby incorporated by reference in its entirety.

### Compositions for use

In some embodiments, the disclosure is directed to a composition of the disclosure for use in inducing ketosis in a non-human mammal in need thereof.

In some embodiments, the disclosure is directed to a use of a composition of the disclosure in the manufacture of a medicament for inducing ketosis in a non-human mammal in need thereof.

Said compositions for use and use for manufacture of a medicament are compatible with the compounds, subjects, doses, formulations, and routes of administration disclosed in the specification.

### EXAMPLES

The disclosure is further illustrated by the following non-limiting examples. The examples serve only for illustration, and are not intended to limit the scope of the disclosure, which is as set out in the claims.

### Example 1

Example 1 provides a method by which compounds of the disclosure were evaluated in a first study, wherein dogs were fasted and the compounds were administered by sublingual gavage.

In a first period, a group of 12 canines (beagles) were administered 5.5% MCT in a first period, and 250 mg/kg C8-BHB in a second period. A 7 day washout period was provided between the first and second periods.

The canines were fasted, and the C8-BHB or MCTs was administered by sublingual gavage.

In each of the first and second periods, serum was sampled prior to administration of the compound, then at minute 15, 30, 45, 60, 75, 90, 120, 180, 240, and 300. Serum was sampled for BHB analysis.

In each of the first, second, third, and fourth period, pathology samples were taken prior to administration of the compound, then at minute 20, 60, 90, 120, 180, 240, and 300.

Where emesis occurred before 60 minutes from dosing, serum BHB content was not analyzed.

Where emesis occurred at or after 60 minutes from dosing and before 300 minutes from dosing, Cₘₐₓ was calculated but AUC was not calculated.

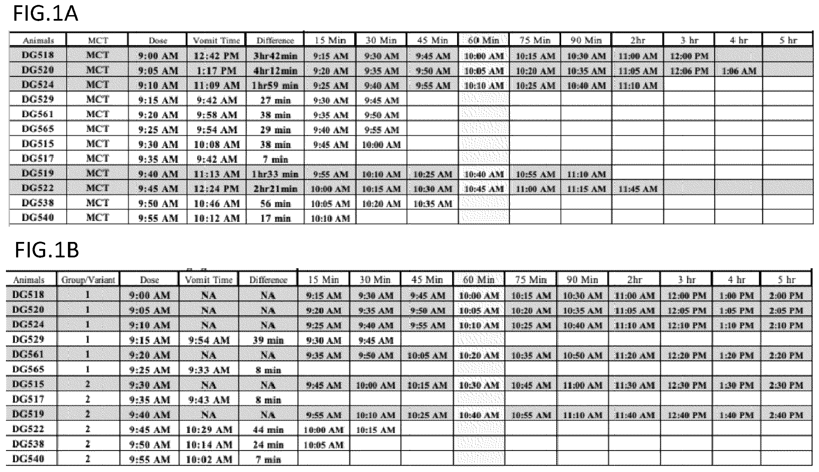

The sampling period data in Figure 1A demonstrates that MCTs were not well tolerated by canines.

The sampling period data in Figure 1B demonstrates that 250 mg/kg C8-BHB was better tolerated by canines compared to MCTs.

Therefore, Example 1 demonstrates that administration of compounds of the disclosure provide improved tolerability compared to prior art methods of inducing ketosis. Compounds of the disclosure provide an improved safety profile compared to known methods for inducing ketosis.

### Example 2

Example 2 demonstrates that administration of compounds of the disclosure (e.g., C8-BHB) induces ketosis and provides an improvement compared to prior art methods administering MCTs.

A group of 6 beagles was evaluated per tested compound of the disclosure. In a first period, the canines were administered 250 mg/kg of C8-BHB. In a second period, the canines were administered 500 mg/kg of C8-BHB. In a third period, the canines were administered MCT at a dose of 5.5%. In a fourth period, the canines were administered 125 mg/kg of C8-BHB. A washout period of 7 days was provided between each of the first, second, third, and fourth periods.

Following the tolerability results of Example 1, the canines were fed, and the compound was administered by gastric gavage in subsequent examples.

In each of the first, second, third, and fourth period, serum was sampled prior to administration of the compound, then at minute 15, 30, 45, 60, 75, 90, 120, 180, 240, and 300. Serum was sampled for BHB analysis.

In each of the first, second, third, and fourth period, pathology samples were taken prior to administration of the compound, then at minute 20, 60, 90, 120, 180, 240, and 300.

If emesis occurred 60 minutes from administration of the compound, serum BHB content was not analyzed.

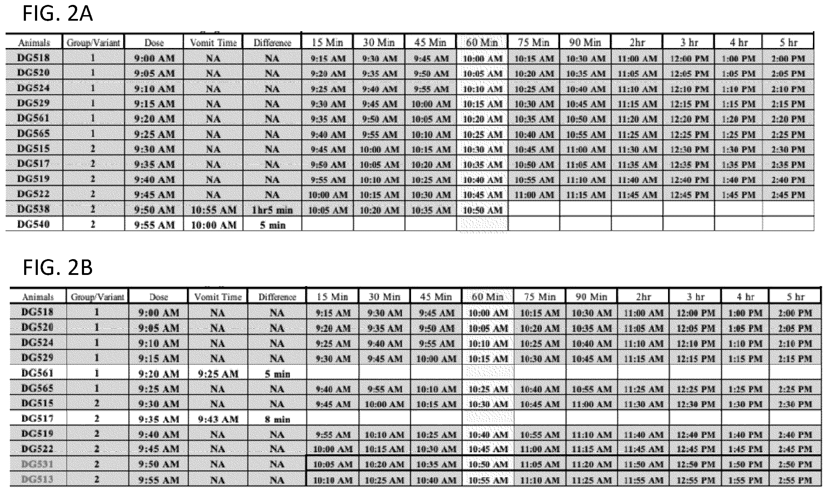

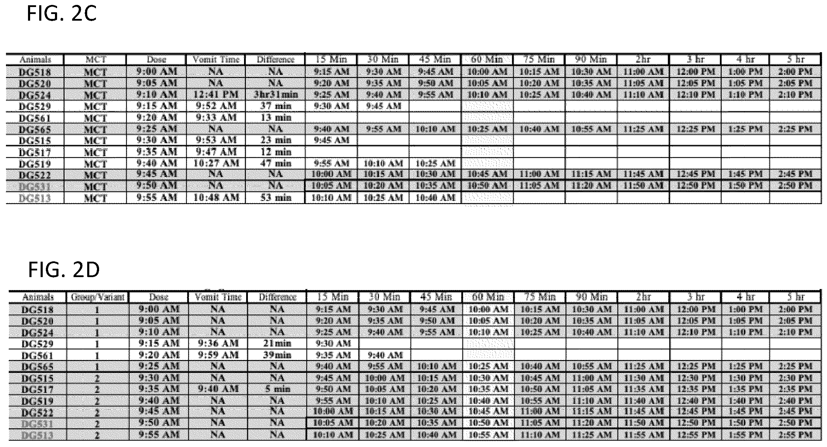

Figures 2A-D demonstrate that the C8-BHB provided improved tolerability compared to MCTs (Figure 2C) at C8-BHB doses of 125 mg/kg (Figure 2D), 250 mg/kg (Figure 2A), and 500 mg/kg (Figure 2B).

Overall, in the fed state, 83% of canines tolerated C8-BHB, compared to only 50% of canines tolerating MCTs.

Therefore, Example 2 demonstrates that administration of compounds of the disclosure results in improved tolerability compared to prior art methods of inducing ketosis. Compounds of the disclosure provide an improved safety profile compared to known methods for inducing ketosis.

### Example 3

Example 3 demonstrates that compounds of the disclosure increase BHB concentration from baseline.

The data obtained from the study of Example 2 was compared to prior art data for MCTs.

C8-BHB and C8x2-BD were selected as exemplary compounds of the disclosure for administration to canines according to the method of Example 2. Each exemplary compound was administered to 6 canines.

Prior art methods achieved a Cₘₐₓ of 0.06 mM following administration of 5.5% MCTs.

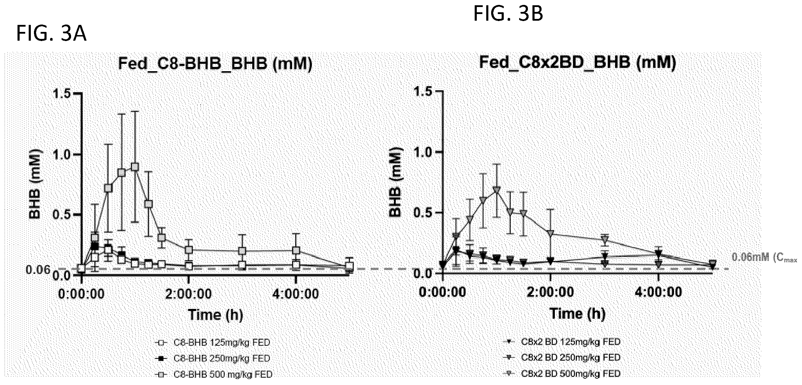

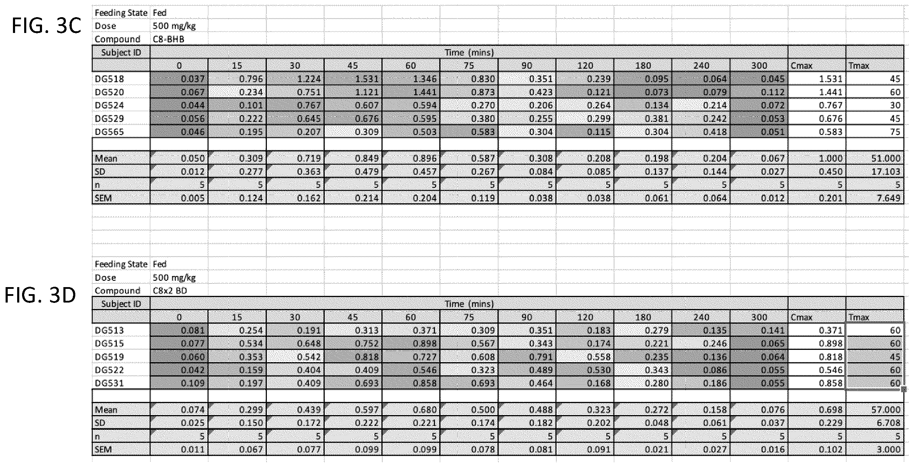

Administration of C8-BHB to canines at doses of 125 mg/kg, 250 mg/kg, and 500 mg/kg induced a significant increase in BHB concentration from baseline, compared to prior art methods (Figure 3A, Figure 3C). For example, administration of 500 mg/kg C8-BHB provided a Cₘₐₓ of 1.00 mM and a Tₘₐₓ of 51 minutes.

Administration of C8x2-BD to canines at doses of 125 mg/kg, 250 mg/kg, and 500 mg/kg induced a significant increase in BHB concentration from baseline, compared to prior art methods (Figure 3B, Figure 3D). For example, administration of 500 mg/kg C8x2-BD provided a Cₘₐₓ of 0.70 mM and a Tₘₐₓ of 57 minutes.

The data depicted in Figures 3A and 3B demonstrate that both exemplary compounds of the disclosure result in an increase in BHB concentration from baseline. All three concentrations result in a significant increase in BHB concentration from baseline compared to the prior MCT study.

This Example demonstrates a significant improvement in BHB concentration relative to baseline - which is a known indicator of inducing ketosis - compared to prior art studies of inducing ketosis by administering MCTs.

### Example 4

Example 4 demonstrates that compounds of the disclosure, as defined by Formula I, represent an improvement over MCTs.

To further evaluate the potential of the compounds of the disclosure for inducing ketosis in canines, the compounds and MCTs were administered to the same canines, as described in Example 2.

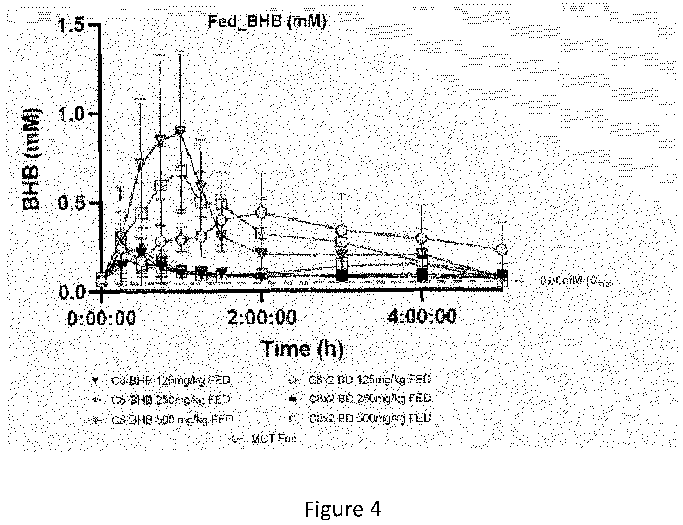

Figure 4 demonstrates the kinetics from the first, second, third, and fourth periods described in Example 2. It demonstrates that both exemplary compounds of the disclosure, C8-BHB and C8x2-BD, increase BHB concentrations to a higher level than MCTs in the same canines at the 500 mg/kg amount. Therefore, the data in Figure 4 demonstrates that compounds of the disclosure provide an improved method of inducing ketosis compared to MCTs.

Figure 4 also demonstrates that the 125 mg/kg and 250 mg/kg amounts of the compounds of the disclosure achieve comparable increases to BHB concentration to MCTs. As described above, compounds of the disclosure demonstrate an improved safety profile compared to MCTs. Therefore, compounds of the disclosure administered to canines in 125 mg/kg and 250 mg/kg amounts also represent an improvement over MCTs.

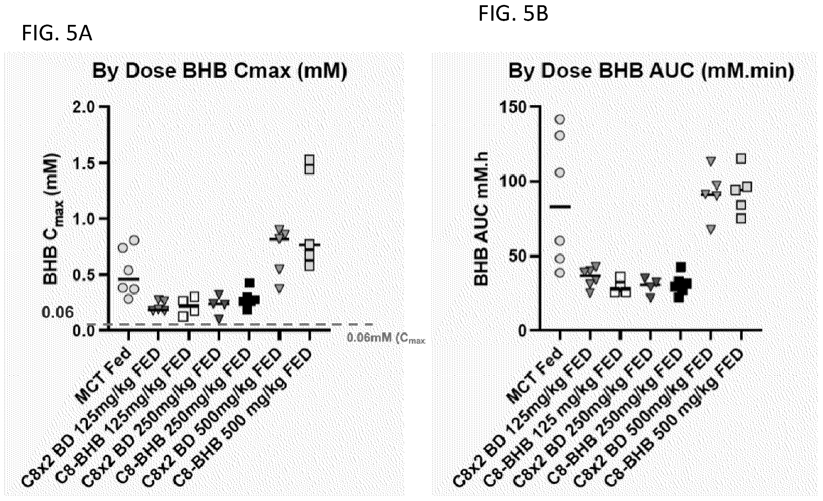

Figure 5A provides the Cₘₐₓ values for the compounds of the disclosure compared to MCTs in the study described in Example 2. It demonstrates that 500 mg/kg of each exemplary compound of the disclosure achieves an improves Cₘₐₓ compared to MCTs. Compounds of the disclosure administered to canines in 125 mg/kg and 250 mg/kg amounts achieve similar Cₘₐₓ values to MCT, while providing an improved safety profile.

Figure 5B provides AUC data for the compounds of the disclosure compared to MCTs in the study described in Example 2. In addition to demonstrating that administration of 500 mg/kg of the compounds of the disclosure produces an increases AUC (mM.min) compared to MCTs, the compounds of the disclosure achieve less variability in AUC compared to the MCT data.

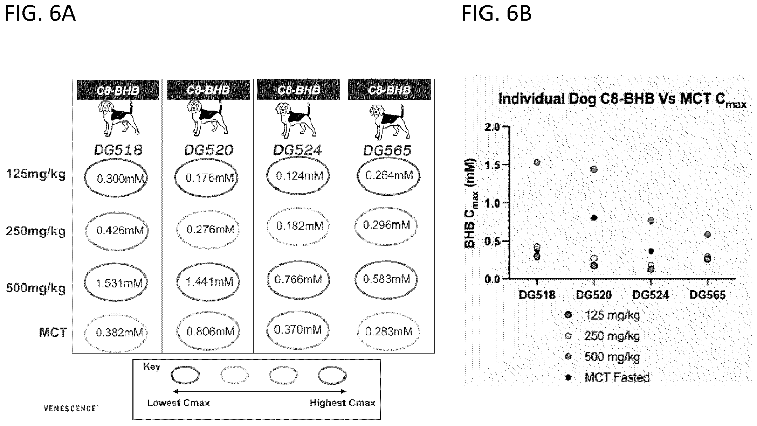

To provide more accurate comparative data, Cₘₐₓ values were compared for the different C8-BHB doses and MCT for individual canines. Figure 6A provides Cₘₐₓ values for four canines to which C8-BHB and MCTs were administered. Said data is further depicted in graphical form in Figure 6B. Figures 6A and 6B demonstrate that two canines showed higher Cₘₐₓ values following administration of an amount of 250 mg/kg of the compound of the disclosure than following administration of MCT when fasted. Therefore, this data further demonstrates that administration of 250 mg/kg of a compound of the disclosure represents an improved method of inducing ketosis compared to MCTs. Moreover, 125 mg/kg shows similar Cₘₐₓ values to MCTs, whilst providing improved tolerability.

Therefore, Example 4 demonstrates that administration of 125 mg/kg, 250 mg/kg, or 500 mg/kg C8-BHB provides an improvement over MCTs.

### Example 5

This example demonstrates that administration of lower amounts of the compounds of the disclosure provides an improvement over prior art methods of administering MCTs.

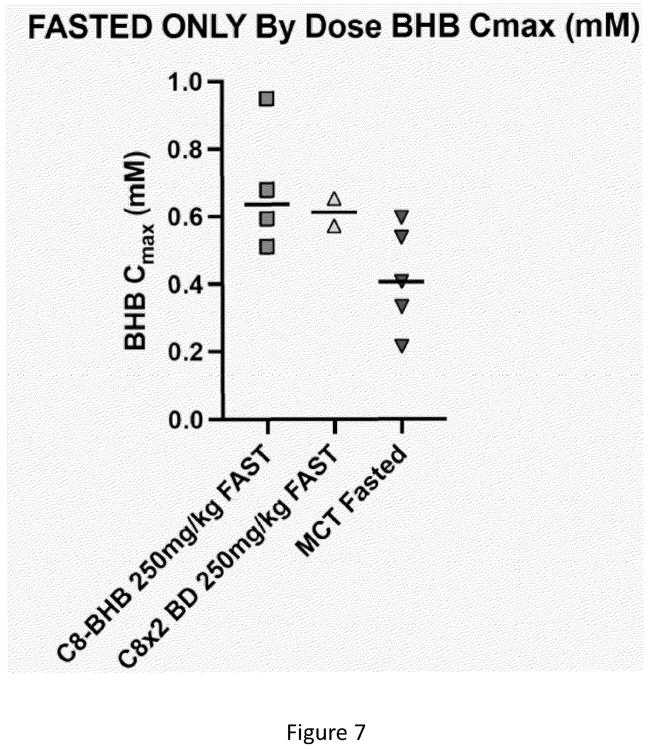

Further analysis of the data from the study of Example 1 on fasted canines is depicted in Figure 7. The data in Figure 7 demonstrates that administration of 250 mg/kg of an exemplary compound of Formula I, C8-BHB, provides improved Cₘₐₓ compared to administration of MCTs.

Therefore, the data in Figure 7 further demonstrates that compounds of the disclosure provide an improvement over MCTs.

### EQUIVALENTS

Those skilled in the art will recognize many equivalents to the specific embodiments of the disclosure escribed herein. The scope of the present disclosure is not intended to be limited to the above Description, but rather is as set forth in the claims.

## Claims

1. A method of inducing ketosis in a non-human mammal in need thereof, comprising administering to the non-human mammal an effective amount of a compound of Formula I: 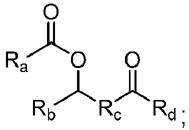 wherein:
Rₐ is unsubstituted or substituted C₄₋₃₀ alkyl;
R_{b} is H, C₁₋₆ alkyl, or substituted alkyl;
R_{c} is CHzHzO or CH₂; and
R_{d} is unsubstituted or substituted C₄₋₃₀ alkyl, or unsubstituted or substituted O-C₄₋₃₀ alkyl;
wherein the amount is from 125 mg/kg to 500 mg/kg.

2. The method of claim 1, wherein the compound is a compound of Formula II: 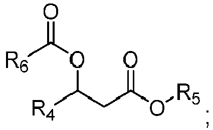 wherein:
R₄ is H, C₁₋₆ alkyl, or substituted alkyl; and
R₅ and R₆ are independently unsubstituted or substituted C₄₋₃₀ alkyl.

3. The method of claim 1, wherein the compound is a compound of Formula III: 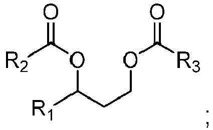 wherein:
R₁ is H, C₁₋₆ alkyl, or substituted alkyl; and
R₂ and R₃ are independently unsubstituted or substituted C₄₋₃₀ alkyl.

4. The method of claim 1, wherein the compound is selected from the group consisting of hexanoyl-ethyl-3-hydroxybutyrate, octanoyl-ethyl-3-hydroxybutyrate, hexanoyl-hexyl-3-hydroxybutyrate, bis-hexanoyl-1,3-butanediol, and bis-octanoyl-1,3-butanediol.

5. The method of any of claims 1-4, wherein the amount of the compound is administered as a food supplement.

6. The method of any of claims 1-5, wherein:
(a) the amount is 125 mg/kg; or
(b) the amount is 250 mg/kg; or
(c) the amount is 500 mg/kg.

7. The method of any one of claims 1 to 6, wherein the amount of the compound is administered at least once per day.

8. The method of any one of claims 1 to 7, wherein the non-human mammal is administered a composition comprising the compound, optionally wherein the composition further comprises one or more compounds or components of a ketogenic diet.

9. A non-human mammal food supplement comprising a compound of Formula I: 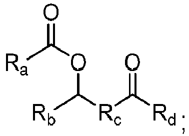 wherein:
Rₐ is unsubstituted or substituted C₄₋₃₀ alkyl;
R_{b} is H, C₁₋₆ alkyl, or substituted alkyl;
R_{c} is CHzHzO or CH₂; and
R_{d} is unsubstituted or substituted C₄₋₃₀ alkyl, or unsubstituted or substituted O-C₄₋₃₀ alkyl;
wherein the amount is from 125 mg/kg to 500 mg/kg.

10. A non-human mammal food comprising a compound of Formula I: 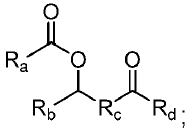 wherein:
Rₐ is unsubstituted or substituted C₄₋₃₀ alkyl;
R_{b} is H, C₁₋₆ alkyl, or substituted alkyl;
R_{c} is CHzHzO or CH₂; and
R_{d} is unsubstituted or substituted C₄₋₃₀ alkyl, or unsubstituted or substituted O-C₄₋₃₀ alkyl;
wherein the amount is from 125 mg/kg to 500 mg/kg.

11. A non-human mammal food additive comprising a compound of Formula I: 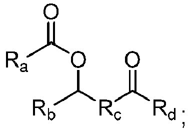 wherein:
Rₐ is unsubstituted or substituted C₄₋₃₀ alkyl;
R_{b} is H, C₁₋₆ alkyl, or substituted alkyl;
R_{c} is CHzHzO or CH₂; and
R_{d} is unsubstituted or substituted C₄₋₃₀ alkyl, or unsubstituted or substituted O-C₄₋₃₀ alkyl;
wherein the amount is from 125 mg/kg to 500 mg/kg.

12. The method of any of claims 1-8, the food supplement of claim 9, the food of claim 10, or the food additive of claim 11, wherein the non-human mammal is a domestic animal.

13. The method, food supplement, food, or food additive of claim 12, wherein the non-human mammal is a companion animal.

14. The method, food supplement, food, or food additive of claim 13, wherein the companion animal is a canine or a feline.

15. The method, food supplement, food, or food additive of claim 14, wherein the non-human mammal is an equine.
